# EUROPEAN PATENT SPECIFICATION

(11) **EP 2 463 272 B1**
(45) Date of publication and mention of the grant of the patent: **28.10.2015**
(21) Application number: 11460061.2
(22) Date of filing: 09.12.2011
(51) Int. Cl.: C07C 263/10, C07C 265/14

(54) **A method of producing toluene diisocyanate (TDI) in the process of the toluene diamine (TDA) phosgenation reaction in the gaseous phase**
Verfahren zur Herstellung von Toluoldiisocyanat beim Verfahren der Toluenediamin-Phosgenierungsreaktion in der Gasphase
Procédé de production de diisocyanate de toluène dans le processus de la réaction de phosgénation de diamine de toluène en phase gazeuse

(30) Priority: 10.12.2010 PL 39321710
(43) Date of publication of application: 13.06.2012
(73) Proprietor: BASF SE, 67056 Ludwigshafen (DE)
(72) Inventor: Baldyga, Jerzy, 05-540 Zalesie Górne (PL); Molga, Eugeniusz, 02-942 Warszawa (PL); Szarlik, Stefan, 05-082 Blizne Laszczynskiego (PL); Wójcik, Wlodzimierz, 01-494 Warszawa (PL); Machniewski, Piotr, 05-825 Grodzisk Mazowiecki (PL); Rudniak, Leszek, 02-506 Warszawa (PL); Piechota, Stanislaw, 03-980 Warszawa (PL); Slawatycki, Arkadiusz, 85-861 Bydgoszcz (PL); Chrupala, Wojciech, 85-704 Bydgoszcz (PL); Lachmajer, Jerzy, 85-149 Bydgoszcz (PL); Ruczynski, Lech, 85-565 Bydgoszcz (PL); Wójcik, Lucjan, 86-801 Niemcz (PL); Stuczynski, Jacek, 85-801 Bydgoszcz (PL)

(56) References cited:
- WO-A1-2008/049783
- US-A1- 2008 146 834
- US-A1- 2010 160 674

## Description

The invention relates to the method for producing toluene diisocyanate (TDI) in the process of toluene diamine phosgenation (TDA) in the gaseous phase, which uses toluene diamine (TDA) contaminated with solids as a substrate.

In recent years, rapid development in the technology of toluene diamine phosgenation in the gas phase can be seen in the process of obtaining toluene diisocyanate (TDI). The new process helps to reduce solvent consumption by about 80% and energy consumption by about 40% compared to the classical method of (TDA) phosgenation in the liquid phase [Urethane chemicals process enters a new phase, 19.08.2008, www.processengineering.co.uk].

The methods known from patent documents, e.g. US 200502729, PL 177910, PL 181030, US 2480089, of conducting the phosgenation of aromatic diamines in the gas phase generally rely on evaporation of diamine, optionally in the presence of an inert gas or vapours of an inert solvent at a temperature of 300 - 400°C. Separately, phosgene is evaporated, sometimes also in a stream of inert gas or vapours of an inert solvent, and subsequently heated to a temperature of 300 - 400°C. The gaseous streams of diamine and phosgene are intensively mixed and directed into the active part of the reactor at a temperature of 300 - 400°C. The process is run in excess phosgene in relation to stoichiometry of reactive amino groups in diamine.

As a result of the reaction, diisocyanate and hydrogen chloride are formed. The reaction mixture is cooled to a temperature higher than the decomposition temperature of the diisocyanate carbamoyl chloride, though allowing condensation of the formed toluene diisocyanate. Cooling of the reaction mixture is frequently carried out directly with an additional stream of solvent at a low temperature. The gas stream remaining after the separation of diisocyanate is separated to isolate the residual diisocyanate, solvent, unreacted phosgene, hydrogen chloride and the inert gas, such as nitrogen. Excess phosgene is recycled to the process.

Phosgenation of toluene diamine (TDA) in the gaseous phase requires feeding gaseous substrates to the reaction zone, i.e. toluene diamine (TDA) and phosgene. Evaporation of toluene diamine (TDA) is a difficult operation. Because of the high boiling point, it requires the use of pure (distilled) diamine and is usually carried out in thin film evaporators at temperatures above the boiling point of the diamine, as described in U.S. patent publication US 20080146834. Often, in the conditions for toluene diamine (TDA) evaporation, by-products are formed that are deposited in the interior of the device/devices used to evaporate toluene diamine (TDA).

The evaporation system should have a short residence time in the evaporator and can be implemented, for example, in a thin film evaporator with a high degree of circulation, in the presence of an inert gas, to prevent thermal degradation of the diamine with the separation of tarry by-products and ammonia.

Another solution for diamine evaporation, in order to prevent thermal degradation, is described in the patent application US 20070043233, in which a special heat exchanger is used that contains milli- or microchannels or tubes through which the separated liquid flows. According to this invention, the heat exchanger's specific surface area is at least min. 1000 m²/m³, and the hydraulic diameter of the channel is 5-10000 µm. However, such a small cross-sections of channels are susceptible to blockage formation and reduction of the heat exchange surface. This increases the frequency of system stoppages and increases the need to clean the evaporator.

US2010/160674 discloses a process for the preparation of TDI in the gas phase, in which problems related to formation of solids are eliminated by controlling the quality of the aromatic diamines used (in particular by keeping the content of ammonia, heavy metals and cycloaliphatic amines low) and by using the diamine evaporation process, wherein at least 0.1 wt. % of the liquid TDA is not vaporized and is not fed into the reactor.

The evaporation of toluene diamine (TDA) in the above systems requires, in every case, the usage of clean, distilled toluene diamine (TDA) that is free of solids.

The subject of the present invention is related to the method of obtaining toluene diisocyanate (TDI) by performing the toluene diamine (TDA) phosgenation reaction in the gaseous phase.

According to the invention, a method of preparing toluene diisocyanate (TDI) in the toluene diamine (TDA) phosgenation in the gas phase relies on the fact that as one of the substrates, non-distilled toluene diamine (TDA) is used, which contains up to 4% by weight of non-volatile substances, and the process of toluene diamine TDA evaporation is carried out in a thin film evaporator with scrapers placed on the rotor, wherein the non-volatile substances in the diamine used and derived from the decomposition of toluene diamine (TDA) in the conditions for carrying out the conversion of the raw material into the gas phase are carried off into a space that is located at the bottom of the evaporator.

Preferably, the non-volatile substances in the diamine used and derived from the decomposition of toluene diamine (TDA) from the space located at the bottom of the evaporator are carried away by a system of cut-off valves located on receiving bunkers for liquid waste or another equivalent system that allows continuous reception of waste without stopping the evaporator.

It is also preferable when in the process of toluene diamine (TDA) evaporation, an inert gas flow is additionally used, wherein any chemical substance can be used as the inert gas, which is a gas under conditions of toluene diamine (TDA) evaporation and does not react with toluene diamine (TDA), specifically nitrogen, dichlorobenzene isomers and mixtures thereof.

It is also preferred, when the inert gas shall be fed concurrently in relation to toluene diamine (TDA) or by means of a lateral supply, whereas the liquid toluene diamine (TDA) is fed from above of the device.

It is also preferable when the inert gas is fed countercurrently in relation to toluene diamine (TDA) or by means of a lateral supply, whereas liquid toluene diamine (TDA) is fed from above the device.

Another advantageous feature is that in the process of toluene diamine (TDA) evaporation, an evaporator with an electric-resistance heating, induction heating or heating by means of organic or inorganic heat-transfer medium is used, including direct exhaust-heating, wherein, preferably, the heating elements are divided into at least 2 sections.

Another advantageous feature is when the inert gas is, most preferably, heated to a temperature equal to the temperature at which evaporation of toluene diamine (TDA) occurs or higher, but not by more than about 200°C.

Preferably, when the measurement at the bottom of the evaporator, to which the non-volatile parts contained in the toluene diamine (TDA) used can flow, is performed using a device with a source of ionising radiation and an ionising radiation detector, such as the Geiger counter.

It is also preferable when the measurement at the bottom of the evaporator, to which the non-volatile parts contained in the toluene diamine (TDA) used can flow, is performed using a device with a source of ionising radiation and an ionising radiation detector, such as the scintillation counter.

Another advantageous feature is when adjustment of the rotational speed of the motor driving the rotor is applied in order to control the process.

Pos. 1 shows schematically the process of producing toluene diisocyanate (TDI) in the process of toluene diamine (TDA) phosgenation, in the gas phase, known in the prior art.

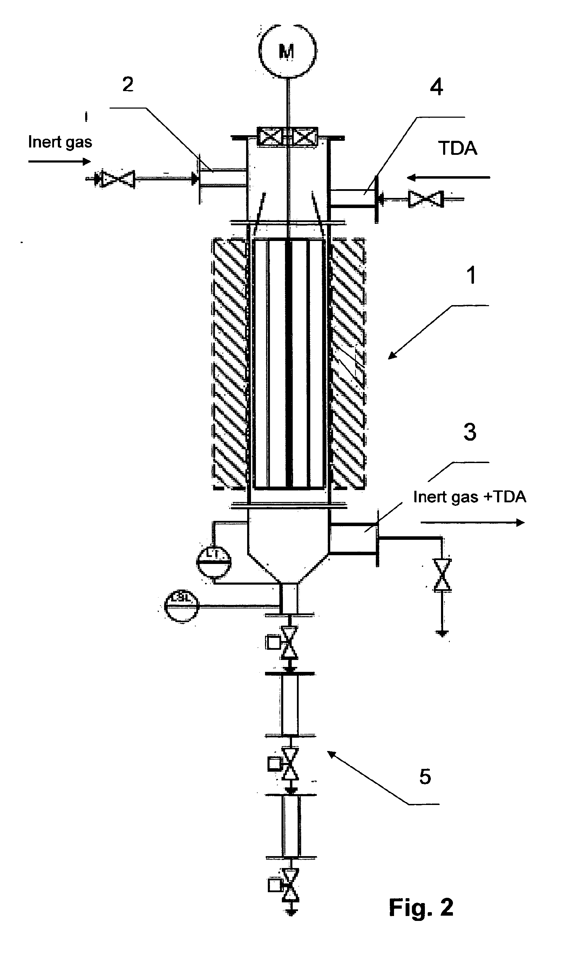

The method of the invention is shown, for example, in the drawing, in which Fig. 1 shows a cross section of a thin-film evaporator with a rotor, to which the inert gas is fed countercurrently, and Fig. 2 shows a cross section of a thin-film evaporator with a rotor, to which the inert gas is fed concurrently, and Fig. 3 shows a system for producing toluene diisocyanate (TDI) in the process of the toluene diamine (TDA) phosgenation reaction in the gaseous phase.

Surprisingly, it was discovered that for the phosgenation process of toluene diamine (TDA) in the gas phase toluene diamine (TDA), containing up to 4% by weight of contaminants, could also be used if a thin-film evaporator is used for the conversion to the gas phase. The contaminants, which, in the conditions of toluene diamine (TDA) evaporation, precipitate on the walls of an evaporator 1, that is on the walls of the device that serves for carrying out the conversion of toluene diamine (TDA) to the gas phase, in the form of deposits of solid particles and tarry bodies are easily removed from the walls of the evaporator by means of scrapers located on the rotating rotor of the evaporator 1. These scrapers can be connected by any means to the rotor and can be made of any material, wherein the shape of the surfaces thereof must ensure removal of non-volatile parts from the surface that is subjected to evaporation, and they must ensure the restoration of the liquid film of toluene diamine (TDA) on the evaporator heating surface. The rotor is set into rotational motion by an electric motor with adjustable speed. The inert gas used to evaporate toluene diamine (TDA) can be introduced into the evaporator 1 by means of the two variants described below.

### Variant 1.

In this variant of the method, molten toluene diamine (TDA) is supplied to the evaporating device, preferably to the thin film evaporator with a rotor, by first upper entry point 4, to which device, by lower entry point 3 the inert gas is countercurrently fed, while overheated to a temperature at which toluene diamine (TDA) evaporation occurs.

### Variant 2.

In this variant of the method, molten toluene diamine (TDA) is supplied to the evaporating device, preferably to the thin film evaporator with a rotor, by first upper entry point 4, to which device, by another upper entry point 2 the inert gas is concurrently fed, while overheated to a temperature at which toluene diamine (TDA) evaporation occurs.

The inert gas, prior to entering the evaporator 1, can be overheated to a temperature higher than the temperature in the evaporator to reduce the amount of heat required in the evaporator 1, but for technical and economic reasons, overheating of the inert gas by more than about 200°C is unprofitable. It is preferable to introduce the inert gas at a temperature equal to the temperature that prevails in the evaporator 1. The inert gas can be introduced into the evaporator 1 in a concurrent or countercurrent manner in relation to the toluene diamine (TDA) flow; it can also be fed by a stub pipe located on the side of the device. Particularly preferable, however, is supplying the inert gas countercurrently by means of the lower entry point 3 of the evaporator 1 in order to obtain rapid mixing of the inert gas with toluene diamine (TDA) vapours and, due to the low toluene diamine (TDA) vapour content in the gas, near the zone of depletion of the liquid toluene diamine (TDA), which guarantees complete evaporation in that zone. However, in every case, the introduction of inert gas and the collection of vapours from the evaporator 1 should ensure a forced flow of gases at the walls of the evaporator 1 in the parts thereof that constitute the effective evaporation surface where evaporation takes place.

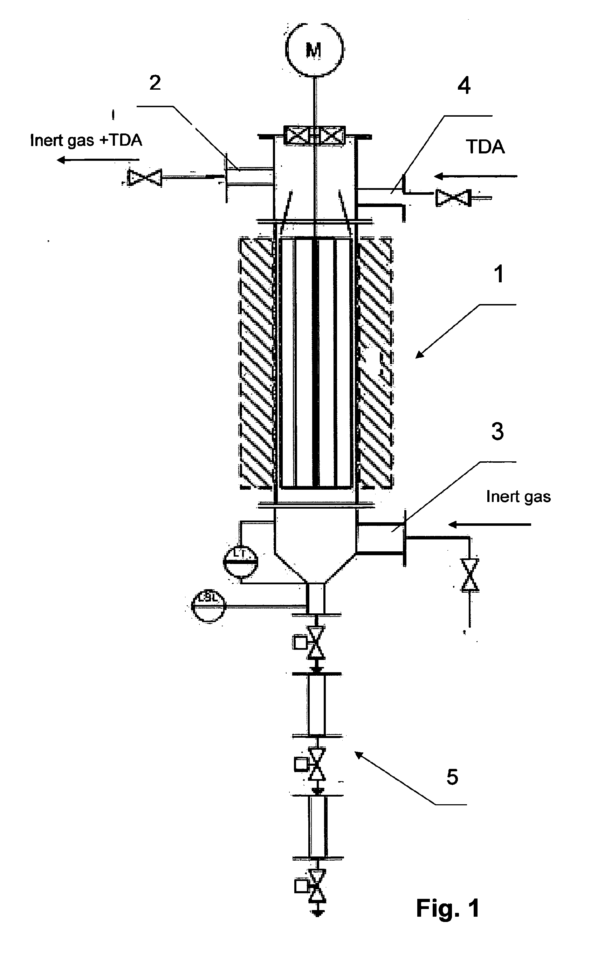

The inert gas, saturated with toluene diamine (TDA) vapours, is carried away from the evaporator by means of the second upper entry point 2 (variant 1 shown in Figure 1), or the inert gas saturated with toluene diamine (TDA) vapours is carried away from the evaporator by means of the lower entry point 3 (variant 2). It is also acceptable to use a different method for carrying away toluene diamine (TDA) vapours in the inert gas. Solids, optionally dissolved in the molten toluene diamine (TDA), flow to the lower part of the evaporator 1 and are carried away from the system. There is no need for circulating evaporated liquid toluene diamine (TDA).

The lower part of the evaporator 1, to which the non-volatile parts contained in the used toluene diamine (TDA) flow, is equipped with a liquid level measurement, wherein for this measurement, an instrument with a source of ionising radiation or an ionizing radiation detector, such as Geiger counter or scintillation counter, can be used.

The evaporator can be heated by means of electric resistance, electric induction or using an organic or inorganic heat-transfer medium, including direct exhaust-heating, while the most preferable method is heating by means of electric resistance or electric induction, due to the low degree of complexity of such a system and ease of temperature control. Preferably, the heating system is divided into several sections with autonomously and automatically controlled temperature from the indications of the temperature sensor clipped on a heated element or, more preferably, located in a hole made in a heated element, at the bottom of which the sensor device is located, and which the bottom is close to the highest degree permitted, for reasons of endurance and corrosion, to the inner surface of the device. The number of sections should be chosen so that the depletion zone of liquid toluene diamine (TDA) overlapped with one or two heating sections while other sections that are responsible for the evaporation, were located above. The division into sections as described above is made so as to minimise overheating parts of the evaporation surface on which depletion of liquid amine occurs, thus minimising the phenomena of amine degradation. It is preferable to use additional 1 or 2 sections below the section of the liquid amine depletion, which allows for periodic enlargement of the evaporation surface and increases productivity.

At the bottom of the evaporator 1, there is installed a system of valves 5 or other appropriate technical solution that allows for removal of non-volatile diamine parts from the evaporator without having to stop the system. The usage of said device under certain conditions allows for the use of toluene diamine (TDA), which, in the manufacturing process, does not need to be distilled for the production process of toluene diisocyanate (TDI), which reduces the costs of toluene diamine (TDA) production. The use of a thin-film evaporator in the operation of evaporating toluene diamine (TDA) allows for the use of an inert gas of lower purity, especially when it comes to oxygen content, as the by-products formed in the oxidation process of toluene diamine are removed along with the non-volatile parts that were contained in the toluene diamine (TDA) used in the process.

As an inert gas, nitrogen can be used or any other chemical substance which is a gas under conditions of toluene diamine (TDA) evaporation that does not react with toluene diamine (TDA), such as dichlorobenzene isomers and mixtures thereof or chlorobenzene. The implementation of the toluene diamine (TDA) evaporation is described in Examples 1 and 2. They provide an illustration of the method without limiting its scope.

### Example 1

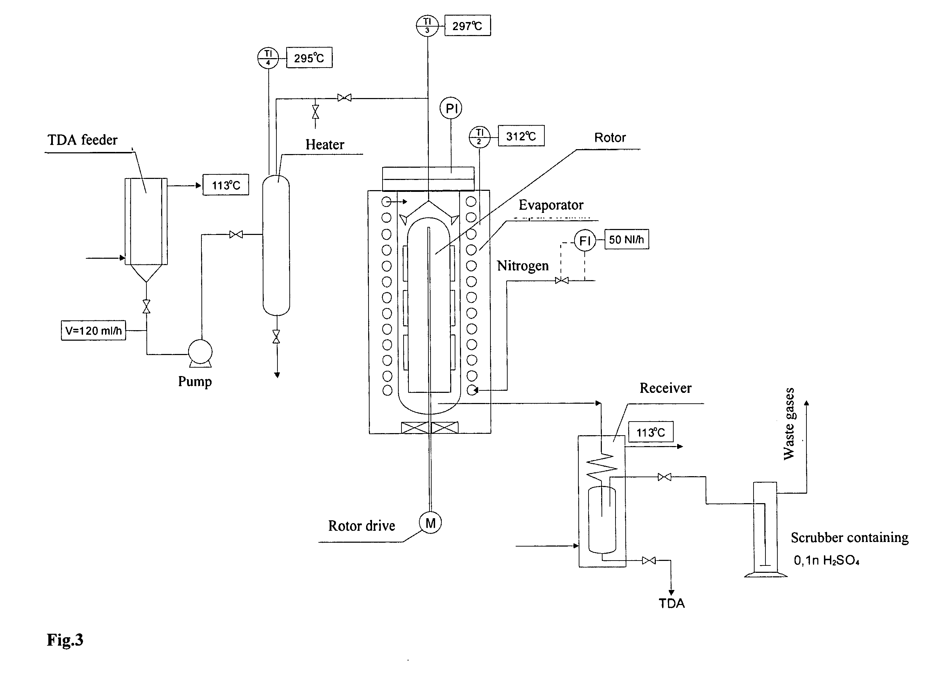

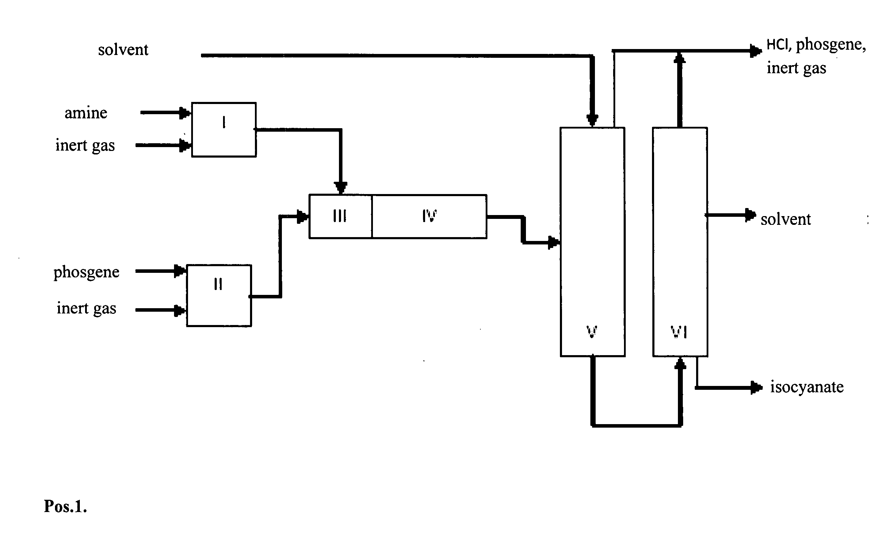

Attempts were made to evaporate toluene diamine (TDA) using the system shown schematically in Figure 3 with a feed of 120 mL/h of molten toluene diamine (TDA), containing 4% by weight of contaminants, which in the conditions of conducting diamine evaporation precipitate as a deposit of solids and tarry bodies.

The nitrogen flow was 50 L/h (as calculated for normal conditions). The temperature of the diamine delivered on the evaporator wall was 295°C. Evaporated toluene diamine (TDA), along with the nitrogen, flowed subsequently through the condenser (capacitor) in which condensation took place. The liquid flowed into a separator, and the effluent nitrogen was passed through a scrubber filled with 150 ml of 0.1 N H₂SO₄. After passing 100 L of nitrogen (calculated at standard conditions), the concentration of acid in the scrubber has been reduced to 0.085 N, which shows that a slight degradation of toluene diamine (TDA) occurred with the discharge of ammonia, which reacted with the sulphuric acid in the scrubber.

The presented data allow for the calculation that during evaporation, approximately 0.03% by weight of dispensed amine has been degraded.

The experiment was conducted for 40 hours, after which the evaporator was disassembled. The evaporator walls were clean, and approximately 190 g of tarry substance, which contained small amounts of toluene diamine (TDA), were collected in the receiver.

The evaporated toluene diamine (TDA) was then subjected to a reaction with gaseous phosgene in conditions similar to the process described in the patent PL 181030, obtaining a yield of toluene diisocyanate (TDI) of 94.5% in relation to pure diamine contained in the raw material, that is going to be subjected to evaporation.

### Example 2

In the same device and under the same temperature and flow conditions, distilled toluene diamine (TDA) was evaporated, of a composition in which there was no non-volatile contaminants that precipitate as a deposit of solids and tarry bodies under the conditions used for diamine evaporation.

After passing through 100 L of nitrogen (calculated at standard conditions), an identical decrease in the concentration of H₂SO₄ was determined in the scrubber through which the inert nitrogen flowed.

After the test, which lasted forty hours, and disassembling the evaporator, no precipitates were detected on the walls of the device, and the amount of tarry liquid at the bottom of the device did not exceed 3 grams.

In the subsequent step of the experiment, the evaporated toluene diamine (TDA) was reacted with the gaseous phosgene to obtain toluene diisocyanate (TDI) with the same efficiency as before, i.e. 94.5%

The given examples show that in the process of toluene diamine (TDA) evaporation, non-distilled toluene diamine (TDA) can be used as a substrate.

Thermal decomposition of toluene diamine (TDA) during the evaporation process at a temperature of 300°C is negligible (approximately 0.03% by weight) and is the same as the toluene diamine (TDA), containing contaminants, which, at the conditions in which the evaporation of toluene diamine (TDA) is carried out, are being discharged in the form of a liquid containing solids and tarry bodies.

## Claims

1. A method for producing toluene diisocyanate TDI in the toluene diamine TDA phosgenation process, in the gaseous phase, **characterised in that** as one of the substrates, non-distilled toluene diamine TDA is used, which contains up to 4% by weight of non-volatile substances, and the process of evaporation of toluene diamine TDA is carried out in a thin film evaporator 1 equipped with scrubbers placed on the rotor, wherein the non-volatile substances contained in the toluene diamine TDA used and derived from the decomposition of toluene diamine TDA in conditions of converting the raw material into the gas phase are carried away into a space located at the bottom of the evaporator 1.

2. A method according to claim 1, **characterised in that** the non-volatile substances contained in the toluene diamine TDA used, and derived from the decomposition of toluene diamine TDA, are carried away from the space located at the bottom of the evaporator by a system of cut-off valves 5 located on the receiving bunkers for liquid waste or another equivalent system that allows for continuous reception of waste without stopping the evaporator 1.

3. A method according to claim 1, **characterised in that** in the process of toluene diamine TDA evaporation, an additional inert gas flow is used, wherein the inert gas can be any chemical substance which is a gas under conditions of toluene diamine TDA evaporation that does not react with toluene diamine TDA, specifically nitrogen, dichlorobenzene isomers and mixtures thereof.

4. A method according to claim 3, **characterised in that** the inert gas shall be fed in a concurrent manner relative to toluene diamine TDA or fed by means of a stub pipe located on the side of the device, while liquid amine is fed from above the device.

5. A method according to claim 3, **characterised in that** the inert gas shall be fed in a countercurrent manner relative to toluene diamine TDA or fed by means of a stub pipe located on the side of the device, while liquid amine is fed from above the device.

6. A method according to claim 1, **characterised in that** in the process of toluene diamine TDA evaporation, the evaporator is used, which utilises heating by means of electrical resistance, induction heating or heating based on using organic or inorganic heat-transfer media, including direct exhaust-heating.

7. A method according to claim 6, **characterised in that** in the process of toluene diamine TDA evaporation, heating elements are used that are subdivided into at least 2 sections.

8. A method according to claim 3, **characterised in that** the inert gas is heated to a temperature equal to the temperature at which evaporation of toluene diamine TDA occurs.

9. A method according to claim 3, **characterised in that** the inert gas is heated to a temperature above the temperature at which evaporation of toluene diamine TDA occurs, but not more than 200°C.

10. A method according to claim 1, **characterised in that** to the measurement of the level of the bottom of the evaporator 1, to which the non-volatile parts can flow, which are contained in the toluene diamine TDA used, the device is used, which contains the source of ionising radiation and the ionising radiation detector, such as a Geiger counter.

11. A method according to claim 1, **characterised in that** to the measurement of the level of the bottom of the evaporator 1, to which the non-volatile parts can flow, which are contained in the toluene diamine TDA used, the device is used, which contains the source of ionising radiation and the ionising radiation detector, such as a scintillation counter.

12. A method according to claim 1, **characterised in that** adjustment of the rotational speed of the motor driving the rotor is applied in order to control the process.

## Patentansprüche

1. Verfahren zur Herstellung von Toluoldiisocyanat TDI beim Prozess der Phosgenierung von Toluoldiamin TDA in der Gasphase, **dadurch gekennzeichnet, dass** als eines der Substrate nicht destilliertes Toluoldiamin TDA verwendet wird, welches bis zu 4 Gew.-% nichtflüchtige Substanzen enthält, und der Prozess der Verdampfung von Toluoldiamin TDA in einem Dünnschichtverdampfer 1, der mit auf dem Rotor angeordneten Schabern ausgestattet ist, durchgeführt wird, wobei die in dem verwendeten Toluoldiamin TDA enthaltenen und aus der Zersetzung von Toluoldiamin TDA stammenden nichtflüchtigen Substanzen unter Bedingungen der Überführung des Ausgangsstoffs in die Gasphase in einen Raum, der sich am Sumpf des Verdampfers 1 befindet, abgeführt werden.

2. Verfahren nach Anspruch 1, **dadurch gekennzeichnet, dass** die in dem verwendeten Toluoldiamin TDA enthaltenen und aus der Zersetzung von Toluoldiamin TDA stammenden nichtflüchtigen Substanzen durch ein System von Absperrventilen 5, die sich an den Aufnahmebunkern für flüssigen Abfall oder einem anderen äquivalenten System, das eine kontinuierliche Aufnahme von Abfall ohne Stoppen des Verdampfers 1 erlaubt, befinden, aus dem Raum, der sich am Boden des Verdampfers befindet, abgeführt werden.

3. Verfahren nach Anspruch 1, **dadurch gekennzeichnet, dass** bei dem Prozess der Verdampfung von Toluoldiamin TDA ein zusätzlicher Inertgasstrom verwendet wird, wobei es sich bei dem Inertgas um eine beliebige chemische Substanz handeln kann, die unter den Bedingungen der Verdampfung von Toluoldiamin TDA gasförmig ist und nicht mit Toluoldiamin TDA reagiert, im Einzelnen Stickstoff, Dichlorbenzol-Isomere und Gemische davon.

4. Verfahren nach Anspruch 3, **dadurch gekennzeichnet, dass** das Inertgas im Gleichstrom in Bezug auf Toluoldiamin TDA zugeführt wird oder mit Hilfe eines Stutzenrohrs, das sich an der Seite der Vorrichtung befindet, zugeführt wird, während flüssiges Amin der Vorrichtung von oben zugeführt wird.

5. Verfahren nach Anspruch 3, **dadurch gekennzeichnet, dass** das Inertgas im Gegenstrom in Bezug auf Toluoldiamin TDA zugeführt wird oder mit Hilfe eines Stutzenrohrs, das sich an der Seite der Vorrichtung befindet, zugeführt wird, während flüssiges Amin der Vorrichtung von oben zugeführt wird.

6. Verfahren nach Anspruch 1, **dadurch gekennzeichnet, dass** bei dem Prozess der Verdampfung von Toluoldiamin TDA der Verdampfer verwendet wird, bei dem das Erhitzen mittels elektrischem Widerstand, Induktionserhitzen oder Erhitzen auf der Basis der Verwendung von organischen oder anorganischen Wärmeübertragungsmedien einschließlich direkter Abgaserhitzung erfolgt.

7. Verfahren nach Anspruch 6, **dadurch gekennzeichnet, dass** bei dem Prozess der Verdampfung von Toluoldiamin TDA Heizelemente verwendet werden, die in mindestens 2 Abschnitte unterteilt sind.

8. Verfahren nach Anspruch 3, **dadurch gekennzeichnet, dass** das Inertgas auf eine Temperatur erhitzt wird, die gleich der Temperatur ist, bei der die Verdampfung von Toluoldiamin TDA stattfindet.

9. Verfahren nach Anspruch 3, **dadurch gekennzeichnet, dass** das Inertgas auf eine Temperatur erhitzt wird, die über der Temperatur liegt, bei der die Verdampfung von Toluoldiamin TDA stattfindet, aber nicht mehr als 200°C.

10. Verfahren nach Anspruch 1, **dadurch gekennzeichnet, dass** zur Messung des Niveaus des Sumpfs des Verdampfers 1, zu dem die nichtflüchtigen Anteile fließen können, die in dem verwendeten Toluoldiamin TDA enthalten sind, die Vorrichtung verwendet wird, die die Quelle von ionisierender Strahlung und den Detektor für ionisierender Strahlung, wie einen Geigerzähler, enthält.

11. Verfahren nach Anspruch 1, **dadurch gekennzeichnet, dass** zur Messung des Niveaus des Sumpfs des Verdampfers 1, zu dem die nichtflüchtigen Anteile fließen können, die in dem verwendeten Toluoldiamin TDA enthalten sind, die Vorrichtung verwendet wird, die die Quelle von ionisierender Strahlung und den Detektor für ionisierender Strahlung, wie einen Szintillationszähler, enthält.

12. Verfahren nach Anspruch 1, **dadurch gekennzeichnet dass** der Prozess durch Einstellung der Rotationsgeschwindigkeit des den Rotor antreibenden Motors gesteuert wird.

## Revendications

1. Procédé pour la production de toluènediisocyanate TDI dans le procédé de phosgénation de toluènediamine TDA en phase gazeuse, **caractérisé en ce qu'**on utilise, comme un des substrats, de la toluènediamine TDA non distillée, qui contient jusqu'à 4% en poids de substances non volatiles, et le procédé d'évaporation de la toluènediamine TDA est effectué dans un évaporateur 1 à film mince équipé de racleurs placés sur le rotor, les substances non volatiles contenues dans la toluènediamine TDA utilisée, et dérivées de la décomposition de la toluènediamine TDA dans des conditions de conversion de la matière première en phase gazeuse, étant évacuées dans un espace situé dans le fond de l'évaporateur 1.

2. Procédé selon la revendication 1, **caractérisé en ce que** les substances non volatiles contenues dans la toluènediamine TDA utilisée, et dérivées de la décomposition de la toluènediamine TDA, sont évacuées de l'espace situé dans le fond de l'évaporateur par un système de clapets de commande 5 situé sur les réservoirs de réception de déchets liquides ou un autre système équivalent qui permet la réception continue de déchets sans arrêter l'évaporateur 1.

3. Procédé selon la revendication 1, **caractérisé en ce que**, dans le procédé d'évaporation de la toluènediamine TDA, on utilise un flux de gaz inerte supplémentaire, le gaz inerte pouvant être n'importe quelle substance chimique qui est un gaz dans les conditions de l'évaporation de la toluènediamine TDA qui ne réagit pas avec la toluènediamine TDA, en particulier l'azote, les isomères de dichlorobenzène et leurs mélanges.

4. Procédé selon la revendication 3, **caractérisé en ce que** le gaz inerte est alimenté à cocourant par rapport à la toluènediamine TDA ou est alimenté au moyen d'une conduite d'échappement située sur le côté du dispositif, alors que l'amine liquide est alimentée à partir du dessus du dispositif.

5. Procédé selon la revendication 3, **caractérisé en ce que** le gaz inerte est alimenté à contre-courant par rapport à la toluènediamine TDA ou est alimenté au moyen d'une conduite d'échappement située sur le côté du dispositif, alors que l'amine liquide est alimentée à partir du dessus du dispositif.

6. Procédé selon la revendication 1, **caractérisé en ce que**, dans le procédé d'évaporation de la toluènediamine TDA, on utilise un évaporateur qui utilise un chauffage au moyen d'une résistance électrique, un chauffage par induction ou un chauffage basé sur l'utilisation d'un milieu de transfert thermique organique ou inorganique, comprenant le chauffage direct par l'échappement.

7. Procédé selon la revendication 6, **caractérisé en ce que**, dans le procédé d'évaporation de la toluènediamine TDA, on utilise des éléments chauffants qui sont sous-divisés en au moins 2 sections.

8. Procédé selon la revendication 3, **caractérisé en ce que** le gaz inerte est chauffé à une température égale à la température à laquelle l'évaporation de la toluènediamine TDA se produit.

9. Procédé selon la revendication 3, **caractérisé en ce que** le gaz inerte est chauffé à une température au-dessus de la température à laquelle l'évaporation de la toluènediamine TDA se produit, mais à pas plus de 200°C.

10. Procédé selon la revendication 1, **caractérisé en ce qu'**on utilise, pour la mesure du niveau du fond de l'évaporateur 1 jusqu'auquel les parties non volatiles, qui sont contenues dans la toluènediamine TDA utilisée, peuvent s'écouler, un dispositif qui contient une source de radiation ionisante et un détecteur de radiation ionisante, tel qu'un compteur Geiger.

11. Procédé selon la revendication 1, **caractérisé en ce qu'**on utilise, pour la mesure du niveau du fond de l'évaporateur 1 jusqu'auquel les parties non volatiles, qui sont contenues dans la toluènediamine TDA utilisée, peuvent s'écouler, un dispositif qui contient une source de radiation ionisante et un détecteur de radiation ionisante, tel qu'un compteur de scintillation.

12. Procédé selon la revendication 1, **caractérisé en ce qu'**on applique un ajustement de la vitesse de rotation du moteur qui entraîne le rotor en vue de réguler le procédé.
